# EUROPEAN PATENT APPLICATION

(11) **EP 4 678 182 A1**
(43) Date of publication of application: **14.01.2026**
(21) Application number: 25188023.3
(22) Date of filing: 08.07.2025
(51) Int. Cl.: A61K 38/39, A61K 9/00, C07K 14/78, C07K 14/00, A61P 17/02, A61P 19/00, C08L 89/06, A61K 38/01

(54) **INJECTABLE PREPARATION BASED ON NATIVE AND DENATURED COLLAGEN AND RELATED PRODUCTION PROCESS**

(30) Priority: 12.07.2024 IT 202400016180
(71) Applicant: Centro Studi Dermatologici S.r.l., 00123 Roma (RM) (IT)
(72) Inventor: CORBO, Andrea, I-00123 ROMA (RM) (IT); FURLAN, Diego, I-00123 ROMA (RM) (IT)
(74) Representative: Fiammenghi, Eva

(57) **Abstract**

Injectable preparation of the reconstitutable type for intradermal injections used in tissue regeneration therapy, wherein the powder bottle contains a combination of native heterologous type I collagen and denatured heterologous type I collagen and wherein the production process comprises the preparatory step (100) of a native collagen (150), the partial hydrolysis step (200) carried out with ultrasound treatment, the drying step (300), the dosing step (400), the packaging step (500), the sterilization step (600) with ionizing rays and finally the preparation step (700) of the second bottle with water for injections.

## Description

### Technical Field

The present patent application for invention relates to the field of medicine in general and more specifically to that of regenerative medicine.

The invention relates to an innovative collagen-based composition capable of stimulating cell renewal.

### Background Art

In humans, as in all mammals, physiological regeneration processes continuously occur, that is, the normal renewal of tissues, in particular the renewal of blood cells, of the superficial layers of the epidermis, of the endometrium during the menstrual cycle, the change of hair and so on. The regeneration process is linked to the replacement of dead cells with cells of the same type, which brings the tissue back to its initial conditions.

A particular role in the field of regenerative medicine is played by collagen, which represents the protein with a structural function, constituting the main component of the extracellular matrix of animal tissue. The term collagen identifies all proteins characterized by a fibrous structure with a right-handed triple helix, elongated and very narrow, measuring 300 nm × 1.5 nm, in which the structural unit is represented by tropocollagen, a macromolecule consisting of the repetition of trimers having a characteristic amino acid composition and in which practically a third of its residues are represented by glycine while another portion of between 15 and 30% is made up of proline and 4-hydroxyproline. Tropocollagen molecules arrange themselves in parallel rows to form fibrils, which may arrange themselves in wavy or parallel bundles to form fibers, and fibers may form bundles of fibers.

Going more specifically into the skin, collagen is produced by fibroblasts in the dermis and, within the dermis, forms a network with another protein called elastin. This structure gives support to the epidermis, keeping the skin toned, compact and elastic.

However, in each individual, collagen production slows down as the years go by. A first step occurs around the age of 25, when the activity of fibroblasts begins to decrease but without there being any visible signs yet. This slowdown leads over time to a deficit in reserves, because the newly produced molecules, called neocollagen, are not sufficient to replace the older molecules, which lose efficiency and are degraded. This is how, already around the age of 30, the signs of aging begin to appear, as well as the first expression lines, which with the passing of time transform into deeper furrows and sagging skin. Currently, the widespread use of collagen as a first choice element in the field of skin regeneration is due, in addition to its role as the main component of the extracellular matrix, also to its excellent biological features and good physical-chemical properties. Specifically in medicine, collagen is widely used with success also in the treatment of chronic lesions, to considerably reduce healing times and in which collagen in the form of sponges or granules of different shapes and sizes, is placed in contact with the lesion and left *in situ* until completely absorbed.

Therefore, its use to prevent, improve and restore skin health is growing rapidly and with a wide spectrum of applications, ranging from the therapeutic to the purely aesthetic.

Several collagen-based products are already known in the art, which are found in various pharmaceutical, medicinal, food and cosmetic products for a wide variety of applications and precisely because of its features of toughness and resistance, the collagen normally used is precisely type I, which as is known represents the basic constituent of the skin, bones and tendons and may be considered as the most abundant species of the various types of collagen.

However, precisely because of the extensive developments in the technique, there is a strong need for new collagen-based formulations that are more effective and allow for more versatile use, as they are aimed at different fields of use.

Therefore, an unavoidable need still exists to advance the prior art by creating an innovative collagen-based composition designed to resolve or at least minimize the above problems, as will be clear from the detailed description of one of its exemplary and non-limiting embodiments, illustrated below.

### Summary of the Invention

The present patent application for an industrial invention is intended to describe and claim a composition as well as the related process, both provided with at least one new and alternative solution to the solutions known so far and in particular the aim is to overcome one or more of the drawbacks or problems referred to above and/or to satisfy one or more needs perceived in the art and in particular deduced from what has been reported above. To this end, the inventors have developed an injectable preparation and the related innovative production process, capable of maximizing the benefits of collagen, while minimizing the side effects thereof. Furthermore, a new collagen-based composition has been created which may be administered not only topically and orally, but also mainly intradermally.

Our invention was born, therefore, from the need to exploit favorably all those regenerative benefits, which derive from the administration of both native collagen and denatured collagen. More specifically, the aim is to exploit heterologous type I collagen as a tissue regenerator. Such assertion derives from the fact that heterologous collagen in its native form, i.e. with its intact triple helix of amino acids in sequence, exerts an important action in the tissue regeneration process, providing a three-dimensional physical structure which favors the migration and settlement of fibroblasts, producers of autologous collagen. But this is only one of the mechanisms for cellular regeneration, another equally important action is exerted by the dipeptides, tripeptides and higher fractions present in denatured collagen, which carry out important biological functions, acting as a substrate for fibroblasts producing endogenous collagen and elastin, as biological messengers and as real growth factors inducing fibroblasts to a marked mitotic and anabolic activity.

Hence the aim of the present invention to combine in a single preparation heterologous type I collagen both in the form of native collagen and in the form of denatured collagen, taking full advantage of all the aforementioned benefits and in any case aimed at an excellent and favorable activity of the product as an efficient regenerator of tissues in general, including the skin.

Therefore, this solution, according to the present invention, constitutes an optimal compromise, since the present invention favorably exploits the known regenerative capacity of heterologous type I collagen in all its currently available forms.

The innovative concept underlying the present invention consists, therefore, in providing for the creation of a completely original product, capable of exploiting in a single product the two properties, that of native collagen and that of denatured collagen and in such a way that both said properties inventively carry out their regenerative actions simultaneously and not in sequence.

Another technical feature which differentiates this injectable preparation based on native and denatured collagen, the object of the present invention, is that it may also constitute a valid means with which to achieve a mesotherapy-type administration, realizing it in the form of an intradermally injectable suspension of a micronized collagen powder.

As regards the starting materials of said inventive composition and more specifically the type of collagen used among the numerous ones existing on the market, these have been selected by the inventors following intense research activity, in order to respond to all the needs which may be deduced both from the prior art and from the stringent requirements represented by the present invention.

In light of all the above-mentioned advantages, the composition in question proves to be an effective and efficient tissue regenerator, particularly indicated in the treatment of skin lesions in general.

Other features of the present invention are described in the following detailed description of one or more specific embodiments thereof, protected by the various dependent claims.

### Brief Description of the Drawings

The previous advantages, as well as other advantages and features of the present invention, will be illustrated by referring to the accompanying figure, which is to be considered purely illustrative and non-limiting or binding for the purposes of the present patent application, in which:

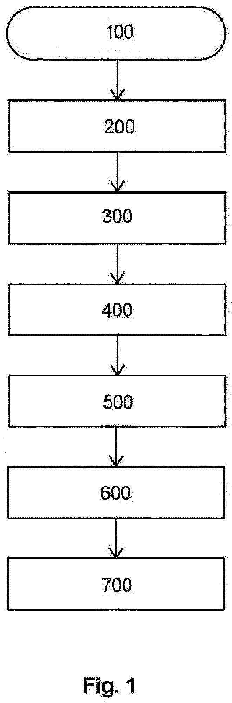

- FIGURE 1 is a flowchart representation of the process according to the present invention.

### Detailed Description of the Invention

The present invention will now be described in detail with reference to the figure and only to some of the possible embodiments, it being possible to describe many others on the basis of the particular technical solutions identified.

A composition is therefore provided comprising an innovative combination of heterologous native type I collagen and heterologous denatured type I collagen, in which said peculiar composition is suitable for carrying out an important action in the tissue regeneration process in general.

Advantageously, said preparation presents itself, compared to existing compounds intended for use in the treatment of tissue regeneration, as a highly valid alternative, safe to administer and with a negligible, if not null, risk of damage from potential side effects. In order to appreciate the particular efficacy that said preparation exhibits, the synergistic effect achieved by the simultaneous administration of both types of heterologous type I collagen, i.e. denatured collagen and native collagen, must be considered, whose benefits in the field of tissue regeneration, as mentioned above, derive from a synergistic and nonsequential action of the properties of both types of collagen.

For the purpose of elucidating the present invention, it must, however, be highlighted that the long polypeptide triple helix of native collagen, placed in contact with body tissue, is split by the enzymatic action of tissue metalloproteases, which reduce said triple helix to polypeptide fragments and even to single amino acids, which together with the polypeptides represent the prevalent components of denatured collagen. It is of interest to note that both the triple helix structure of native collagen and the polypeptide fragments derived from it as well as the amino acids, play different roles, but all aimed at the fundamental process of tissue regeneration and that therefore said roles do not occur simultaneously but sequentially depending on the degree of proteolysis carried out on the native collagen during tissue application. The present invention, through said inventive association, tends to advantageously and simultaneously optimize all the properties of the individual compounds, implementing an injectable preparation that is particularly favorable in the tissue regeneration process. A preferred embodiment, according to the present invention, provides that said tissue regeneration process is aimed at the regeneration of skin tissue.

A preferred embodiment, although the administration may take place in the form of any preparation which may be taken orally or by skin absorption, relates to an administration carried out by intradermal injections aimed at a mesotherapy therapy, consisting of a reconstitutable preparation made up of an association in the form of a powder of heterologous type I collagen, i.e. denatured collagen and native collagen and a solvent for injectable preparations.

Therefore, in light of the results of previous research, within the scope of the definition of the present invention, further studies have been conducted aimed at obtaining the particularly effective inventive association, experimenting with new techniques to obtain an injectable atomized product, with original chemical-physical features due to the use of percentages of partially denatured collagen, so as to make native and denatured collagen coexist within the same preparation.

Regarding the choice of collagen, as a raw material, to be used in the preparatory process shown in Fig. 1, this was not trivial. The starting hypothesis was to use in the preparatory step 100 a native collagen 150 of animal origin, preferably bovine, with a collagen content of at least 99% by weight and in which said native collagen 150 has a microbiological purity close to sterility, i.e. with a bacterial load content constantly below the value of 10 CFU/g and free from potential contamination. In said preparatory step 100, the native collagen 150 is transformed into a dense collagen gel 180, by adding water and HCl up to a pH between 2.5 and 3 and preferably equal to 2.8 and in which said collagen gel 180 has a collagen content equal to 12-15% by weight.

The subsequent step 200 of partial hydrolysis of collagen, in which the aforementioned collagen gel 180 from the previous step is subjected to partial hydrolysis, comprising a dilution with water of said collagen gel 180 and then passing it through a steel mini-reactor where an ultrasound treatment is carried out which, by causing high-frequency oscillations of pressure and depression, is able to cause consequent cavitation phenomena, demolishing in a controlled manner and up to a desired degree of hydrolysis the collagenic structure of the collagen gel 180 according to a scheme which involves multiple passages in said mini-reactor and until obtaining a mixed native and denatured collagen gel 190 with a desired degree of hydrolysis.

The next step consists of a drying step 300 by subjecting said mixed collagen gel 190 to a common spray-drying technique, through which a mixed collagen powder 350 is obtained, composed as a whole of denatured heterologous type I collagen and native heterologous type I collagen.

The following dosing step 400, in which said mixed collagen powder 350 is fractionated using a common powder micro-dosing device.

For strictly descriptive and non-limiting purposes of the present invention, it should be highlighted that the weight ratio between the heterologous type I collagen and the native type I collagen, in said mixed collagen powder 350 is between 10:90 and 90:10 and preferably equal to 50:50.

The next packaging step 500 of said mixed collagen powder 350 which is carried out in airtight glass bottles.

The next sterilization step 600 of said mixed collagen powder 350 with ionizing rays. The next preparation step 700 of the second bottle with water for injections or 0.9% NaCl saline solution.

In this way, a product is obtained consisting of a reconstitutable preparation made up of a bottle of mixed collagen powder 350 and a bottle of solution for injections.

A preferred embodiment according to the present invention consists in that said injectable preparation based on native collagen and denatured collagen is suitable for use in the medical treatment of mesotherapy.

It should, however, be highlighted that, although said inventive injectable preparation is aimed at tissue regenerative use and may effectively be aimed at tissue treatment in the dermatological field, it should not be limited to this, as it may effectively include tissue regeneration in the ophthalmological field, in the gynecological field, in the orthopedic field, in diabetology, gerontology and angiology, but also in dermatology and dentistry. Although such an invention has been described with reference to the specific and concrete embodiment shown in the present document, it must not be considered limited to the details indicated and the patent must be considered inclusive of the modifications and changes, which may derive from the following claims. In fact, the data provided herein are purely illustrative and absolutely non-limiting of the scope of the present invention, as they serve exclusively to enable a person skilled in the art to understand some possible applications and embodiments of the invention. Therefore, several obvious modifications could clearly be made by the average person skilled in the art to the previous exemplary and non-limiting embodiments described with reference to the figures, without this implying an extension beyond the inventive concept, which is the basis of the present invention, as defined by the following claims.

## Claims

1. Injectable collagen-based preparation **characterized in that** said injectable preparation being of the reconstitute type, it consists of a bottle of mixed collagen powder (350) and a bottle of solution for injectable preparations, **in that** said mixed collagen powder (350) is an association of native heterologous type I collagen and denatured heterologous type I collagen and **in that** in said mixed collagen powder (350) the weight ratio between the heterologous type I collagen and the native type I collagen is between 10:90 and 90:10 and preferably equal to 50:50.

2. Injectable collagen-based preparation according to any one of the preceding claims, **characterized in that** said preparation being used for intradermal injections, said preparation is intended for mesotherapy therapy.

3. Injectable collagen-based preparation according to any one of the preceding claims, **characterized in that** it is intended for tissue regeneration treatment.

4. Injectable collagen-based preparation according to the preceding claim 3, **characterized in that** it is intended for regeneration treatment in the dermatological field.

5. Injectable collagen-based preparation according to any one of the preceding claims 1 to 3, **characterized in that** it is intended for treatment in the dental field.

6. Injectable collagen-based preparation according to any one of the preceding claims, **characterized in that** it is intended for treatment for tissue regeneration in the orthopedic field.

7. Production process of an injectable collagen-based preparation according to any one of the preceding claims, **characterized in that** it comprises the following steps in succession, **in that** the preparatory step (100) consists in the transformation of a native collagen (150) into a dense collagen gel (180) by the addition of water and HCl up to a pH between 2.5 and 3 and preferably equal to 2.8, **in that** the partial hydrolysis step (200) of the collagen consists in the dilution of said collagen gel (180) with water, in the passage of said diluted collagen gel (180) in a steel mini-reactor where an ultrasound treatment is carried out capable of causing high-frequency oscillations of pressure and depression suitable for causing cavitation phenomena, wherein said treatment demolishes in a controlled manner until said collagen gel (180) with a desired degree of hydrolysis is obtained and wherein said collagen gel (180) is passed multiple times in said mini-reactor and until a mixed collagen gel (190) with a desired degree of hydrolysis is obtained, **in that** the drying step (300) is carried out by subjecting said mixed collagen gel (190) to a common spray-drying technique, through which a mixed collagen powder (350) is obtained composed of a combination of denatured heterologous type I collagen and native heterologous type I collagen, the dosing step (400) of said mixed collagen powder (350) which is carried out by using a common micro-dosing device for powders, the packaging step (500) of said mixed collagen powder (350) which is carried out in hermetic glass bottles, the sterilization step (600) of said mixed collagen powder (350) with ionizing rays and finally the preparation step (700) of the second bottle with water for injectable preparations.

8. Production process of the collagen-based injectable preparation according to claim 7, **characterized in that** said native collagen (150) being of bovine origin, the title of said native collagen (150) is equal to 99% by weight, **in that** said collagen gel (180) has a collagen title equal to 12-15% by weight, and **in that** said native collagen (150) has a bacterial load content lower than the value of 10 CFU/g.
